(19) Europäisches Patentamt
European Patent Office
Office européen des brevets

(11) Numéro de publication : **0 019 517**
**B1**

(12) # FASCICULE DE BREVET EUROPÉEN

(45) Date de publication du fascicule du brevet : **19.05.82**

(51) Int. Cl.³ : **C 07 C 118/06**, C 07 C 119/048, B 01 J 31/18

(21) Numéro de dépôt : **80400605.4**

(22) Date de dépôt : **06.05.80**

(54) **Procédé de préparation des isocyanates organiques à partir des dérivés nitrés.**

(30) Priorité : **15.05.79 FR 7912272**

(43) Date de publication de la demande : **26.11.80 (Bulletin 80/24)**

(45) Mention de la délivrance du brevet : **19.05.82 Bulletin 82/20**

(84) Etats contractants désignés : **BE CH DE FR GB IT LI LU NL SE**

(56) Documents cités :
**FR - A - 2 014 174**
**FR - A - 2 032 277**
**US - A - 3 935 247**
**US - A - 3 978 137**

(73) Titulaire : **P C U K PRODUITS CHIMIQUES UGINE KUHLMANN**
**Service Propriété Industrielle Tour Manhattan**
**F-92087 Paris La Défense 2 Cedex 21 (FR)**

(72) Inventeur : **Cognion, Jean-Marie**
**85, Route de Charly**
**F-69230 Saint Genis Laval (FR)**
Inventeur : **Kervennal, Jacques**
**134, rue Docteur Locard**
**F-69005 Lyon (FR)**

(74) Mandataire : **Foiret, Claude et al**
**P C U K Produits Chimiques Ugine Kuhlmann Service Propriété Industrielle Tour Manhattan - Cedex 21**
**F-92087 Paris la Défense 2 (FR)**

**EP 0 019 517 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation des isocyanates organiques à partir des dérivés nitrés

La présente invention concerne un procédé de synthèse des isocyanates organiques à partir des composés nitrés et plus particulièrement un procédé de préparation en phase liquide des isocyanates aromatiques par réaction entre les dérivés nitrés aromatiques et le monoxyde de carbone en présence de complexes métalliques des porphyrines préparés in situ dans le milieu de réaction.

Les isocyanates aromatiques sont des intermédiaires organiques de grand intérêt. Deux d'entre eux sont particulièrement importants industriellement. Il s'agit du toluène diisocyanate-2,4 et du diphénylméthane diisocyanate-4,4' qui sont utilisés dans la synthèse des polyuréthanes. Les procédés industrialisés pour la préparation de ces produits font intervenir la réaction de phosgénation d'une amine provenant de l'hydrogénation catalytique d'un dérivé nitré. Les inconvénients de ces procédés sont multiples : ils nécessitent la synthèse et la manipulation de phosgène, produit très dangereux ; ils produisent de l'acide chlorhydrique en quantités importantes ce qui exige l'implantation et l'entretien coûteux d'un atelier particulier pour l'électrolyse de cet acide afin de recycler le chlore.

L'intérêt que présente un procédé évitant l'utilisation du phosgène est évident et plusieurs brevets décrivent des compositions catalytiques permettant de réaliser, à température et pression élevées, la synthèse d'isocyanates par réaction d'un composé organique nitré avec l'oxyde de carbone. C'est ainsi que le brevet français n° 1 600 529 décrit l'utilisation comme catalyseur d'un halogénure de métal noble en présence d'une base aminée à caractère aromatique ; le brevet allemand n° 1 910 303 décrit des catalyseurs formés d'halogénures ou d'oxydes de Ru, Rh, Pd, Os, Ir, Pt et d'un composé soufré hétéroaromatique en présence éventuellement d'un oxyde de Cr, Mo, Nb, W, V ; le brevet français n° 1 567 321 décrit l'utilisation d'un système catalytique formé d'un halogénure de métal noble et d'un composé organique du phosphore, par exemple une triarylphosphine ou un phosphite. Le brevet français n° 2 155 242 décrit des systèmes catalytiques constitués d'un ou plusieurs halogénures de palladium et/ou de rhodium, d'une ou plusieurs bases azotées hétéroatomiques et d'un co-catalyseur formé d'un ou plusieurs borates de fer ; dans le brevet français n° 2 120 110, la formulation catalytique comprend outre un halogénure de palladium et des bases azotées hétéroatomiques, un co-catalyseur constitué d'un ou plusieurs molybdates de fer et/ou de manganèse. Tous ces systèmes produisent des isocyanates à partir de composés nitrés avec des sélectivités et des productivités variables.

Dans sa demande de brevet français n° 78 06927, la demanderesse décrit l'utilisation de métalloporphyrines comme catalyseurs de la réaction de carbonylation des dérivés nitrés aromatiques en isocyanates. Selon cette demande de brevet n° 78 06927, on fait réagir les dérivés nitrés aromatiques et le monoxyde de carbone en présence du catalyseur préalablement préparé par réaction d'un sel ou d'un complexe métallique sur un ligand porphyrine, lui-même préparé suivant les modes de synthèse cités et décrits dans les ouvrages de K.M. SMITH, Porphyrins and Metalloporphyrins, Elsevier (1975), et de D. DOLPHIN, The Porphyrins, vol. 1 Academic Press (1978).

La présente invention permet de simplifier les conditions opératoires de cette synthèse des isocyanates en évitant la préparation initiale des complexes métalloporphyrines, ces derniers se formant in situ en cours de réaction. Le procédé de synthèse des isocyanates aromatiques est ainsi caractérisé en ce qu'on fait réagir le dérivé nitré aromatique avec le monoxyde de carbone en présence d'un catalyseur constitué de porphyrine métallique synthétisée in situ à partir d'une porphyrine et d'un métal ou d'un dérivé métallique.

Eventuellement ce procédé permet non seulement de fabriquer les isocyanates, mais encore de former les complexes métalloporphyrines sous pression de monoxyde de carbone en présence d'un dérivé nitré, ces complexes pouvant être récupérés pour d'autres emplois.

Les métalloporphyrines ainsi formées ont une bonne stabilité chimique et thermique ; ils permettent d'effectuer avec de bons rendements la réaction de carbonylation directe des dérivés nitrés aromatiques en isocyanates en évitant notamment la formation des dérivés azo.

Selon l'invention, le composé nitré est mis en contact avec de l'oxyde de carbone à température et pression élevées en présence d'une porphyrine et d'un ou plusieurs dérivés métalliques. Il est possible d'opérer en présence d'un solvant organique, selon une technique discontinue dans un appareillage de type autoclave ou selon une technique continue permettant d'éliminer dès sa formation l'isocyanate produit selon l'équation de réaction :

$$R-NO_2+3\ CO \xrightarrow[\text{(cata)}]{} RNCO+2\ CO_2$$

Le procédé selon l'invention est applicable aux composés aromatiques comportant un ou plusieurs groupements nitrés fixés sur un atome de carbone d'un noyau aromatique, ces composés pouvant être représentés par la formule générale :

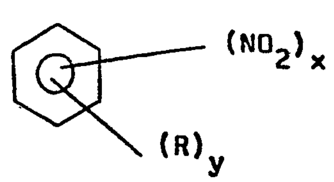

dans laquelle X = 1 ou 2 et y = 0, 1, 2, 3, R étant un groupement d'atomes ou un atome fixé sur le noyau aromatique et pouvant représenter un groupement alkyle ayant de 1 à 10 atomes de carbone, un atome d'halogène, chlore ou brome par exemple, ou un groupement alkoxy OR' dans lequel R' est un radical alkyle ayant de 1 à 10 atomes de carbone. Des exemples, non limitatifs, de composés aromatiques à une ou plusieurs fonctions nitrées, utilisables selon l'invention, sont le nitrobenzène, l'orthonitrotoluène, le paranitrotoluène, le dinitro-1,2 benzène, le dinitro-1,3 benzène, le dinitro-1,4 benzène, le dinitro-2,4 toluène, le dinitro-2,6 toluène, le méthoxy-1 dinitro-2,4 benzène, le chloro-1 nitro-2 benzène, le chloro-1 dinitro-2,4 benzène.

Les ligands porphyrines utilisés peuvent porter différents substituants sur le cycle carboné, par exemple un ou plusieurs groupements alkyles linéaires ou ramifiés comportant de 1 à 10 atomes de carbone, des groupements aromatiques comportant de 6 à 12 atomes de carbone, des groupements aromatiques comportant de 6 à 12 atomes de carbone, un ou plusieurs groupes chlorures d'acide, amides ; cependant, on utilise de préférence la tétraphénylporphyrine et l'octaéthylporphyrine.

La réaction peut être conduite en l'absence de solvant, mais la présence d'un solvant dans le réacteur favorise généralement la sélectivité en isocyanates. Les solvants utilisés de préférence sont les hydrocarbures saturés linéaires ou ramifiés comportant de 6 à 12 atomes de carbone et préférentiellement le décane, la décaline ou les solvants aromatiques tels que le benzène, le toluène ou le xylène ou bien les halogénures aromatiques tels que le chlorobenzène et les dichlorobenzènes. La proportion de solvant n'est pas critique mais on opère en général avec des solutions contenant de 5 % à 50 % en poids du dérivé nitré dans le solvant.

Selon l'invention on introduit dans le réacteur le composé nitré, le solvant de réaction, la porphyrine et le dérivé métallique habituellement sous forme de poudre métallique, d'un oxyde ou d'un sel, de préférence chlorure ou acétate. Après balayage à l'azote, on place le réacteur sous pression de monoxyde de carbone et chauffe jusqu'à la température de réaction. En fin de réaction, on récupère le mélange global dans lequel on peut vérifier par spectroscopie visible la présence de la métalloporphyrine formée in situ. On distille le solvant et l'isocyanate formés et obtient un résidu contenant le complexe qui peut être recyclé tel quel ou auparavant purifié, par exemple par passage sur une colonne remplie d'alumine en utilisant du chloroforme comme solvant d'élution ou en utilisant une technique d'extraction par un solvant tel que l'éther. La métalloporphyrine peut ainsi être obtenue avec un rendement de 98 % par rapport au métal introduit. Le recyclage du catalyseur se fait sans perte d'activité.

Le métal ou le dérivé métallique qui convient particulièrement pour l'invention est choisi parmi les métaux ou les dérivés des métaux des groupes VIII et $I_B$ de la classification périodique des éléments et notamment les suivants : fer, ruthénium, rhodium, palladium, iridium, cuivre et plus particulièrement rhodium et palladium.

La concentration en métal introduit exprimée par le rapport entre le nombre d'atomes-grammes du métal et le nombre de groupements nitrés à transformer peut varier entre $10^{-4}$ et 1 et préférentiellement entre $5 \cdot 10^{-3}$ et $10^{-1}$.

La teneur en porphyrine introduite peut varier entre $10^{-1}$ et 10 moles par atome-gramme de métal, mais on préfère utiliser la quantité stœchiométrique nécessaire pour former le complexe.

Les températures de réaction sont comprises entre 100 °C et 500 °C et plus particulièrement entre 150 °C et 300 °C suivant la nature et la stabilité des réactifs mis en jeu dans les conditions opératoires.

Les pressions de réaction sont comprises entre 20 et 500 bars, de préférence entre 150 et 350 bars, et doivent être suffisantes pour maintenir en phase liquide une fraction importante des réactifs et introduire une quantité totale d'oxyde de carbone correspondant à un rapport molaire CO/groupement $NO_2$ généralement compris entre 3 et 100 et de préférence entre 10 et 65.

Les essais décrits dans les exemples ci-après ont été réalisés en discontinu en autoclave de 500 ml ou 300 ml, muni d'un dispositif d'agitation magnétique, pouvant opérer sous des pressions allant jusqu'à 500 bars et des températures de 300 °C. L'autoclave, chargé avec les différents réactifs en solution, est balayé à l'azote avant d'être mis sous pression d'oxyde de carbone à température ordinaire. L'autoclave, isolé, est alors chauffé à la température choisie et l'avancement de la réaction est contrôlé par enregistrement de la pression.

Après réaction on laisse refroidir l'autoclave, décompresse et les analyses sont effectuées par dosage chimique, chromatographie liquide par perméation de gel et chromatographie en phase vapeur.

Les résultats indiqués s'entendent pour les définitions suivantes :

— T.T.G., le taux de transformation globale =

$$\frac{\text{Nombre de moles de dérivé nitré transformé}}{\text{Nombre de moles de dérivé nitré introduit}} \times 100$$

— Sélectivité en isocyanate =

$$\frac{\text{Nombre de moles d'isocyanate formé}}{\text{Nombre de moles de dérivé nitré transformé}} \times 100$$

— Rendement en isocyanate =

$$\frac{\text{Nombre de moles d'isocyanate formé}}{\text{Nombre de moles de dérivé nitré introduit}} \times 100$$

### Exemple 1

On charge dans l'autoclave 10 g de nitrobenzène, 0,6 g de tétraphénylporphyrine et 0,1 g de noir de palladium. On complète le volume total à 100 ml à l'aide d'orthodichlorobenzène puis on ferme l'autoclave. Après balayage à l'azote on introduit 200 bars de monoxyde de carbone puis on chauffe à 240 °C. On laisse ainsi réagir pendant 8 heures tout en maintenant une agitation, puis refroidit et analyse le mélange. Le spectre d'absorption optique dans le visible, effectué sur une dilution dans le chloroforme du mélange brut de réaction, montre que la porphyrine de palladium s'est bien formée dans l'autoclave ; on obtient, en effet, un spectre à deux bandes caractéristiques d'une métalloporphyrine avec une bande d'absorption à 524 nm et une bande intense, la « bande de Soret », à 418 nm. La tétraphénylporphyrine possède, elle, un spectre différent avec des maximas d'absorption à 645 nm, 592 nm, 550 nm, 513 nm et la « bande de Soret » à 419 nm.

Le T.T.G. du nitrobenzène est de 100 % et la sélectivité en phénylisocyanate de 76,5 %. On ne détecte pas d'azobenzène.

### Exemple 2

Après distillation du phénylisocyanate formé et de l'orthodichlorobenzène contenus dans le mélange réactionnel de l'exemple 1 on place le résidu obtenu, qui contient la tétraphénylporphyrine de palladium formée, dans l'autoclave. On introduit ensuite 7 g de nitrobenzène et complète à 100 ml le volume total à l'aide d'orthodichlorobenzène. On balaye à l'azote et introduit 200 bars de CO. On isole l'autoclave et en maintenant une agitation on chauffe à 240 °C pendant 3 heures. Après refroidissement on analyse le mélange. Le T.T.G. du nitrobenzène est de 94,7 % et la sélectivité en phénylisocyanate de 53 %. On ne détecte pas d'azobenzène.

### Exemple 3

On opère comme dans l'exemple 1, mais en doublant les quantités de noir de palladium et de tétraphénylporphyrine. Après réaction, on distille le phénylisocyanate et le solvant et dissout le résidu, contenant la porphyrine de palladium, dans du chloroforme. On chromatographie alors la solution dans une colonne remplie d'alumine neutre en éluant avec du chloroforme. On recueille ainsi une solution rouge foncée qu'on évapore lentement à sec. Le précipité obtenu est identifié comme étant de la tétraphénylporphyrine de palladium pure par spectroscopie visible, spectroscopie de masse et microanalyse. Le rendement en complexe est de 91 %.

On charge dans l'autoclave 0,7 g de ce complexe, 10 g de nitrobenzène et complète à 100 ml le volume total par de l'orthodichlorobenzène. Après balayage à l'azote, on introduit 200 bars de CO, isole l'autoclave et le porte à 240 °C sous agitation pendant 6 heures. Après refroidissement on analyse le mélange. Le T.T.G. du nitrobenzène est de 95,3 % et la sélectivité en phénylisocyanate de 62 %. On traite de la même façon que ci-dessus le catalyseur, ce qui permet de vérifier que la porphyrine de palladium formée ne se dégrade pas ; on l'obtient à nouveau avec un rendement de 90 % par rapport au complexe introduit.

### Exemple 4

On place dans l'autoclave 10 g de nitrobenzène, 0,6 g de tétraphénylporphyrine et 0,1 g de noir de palladium puis complète à 100 ml le volume total à l'aide d'orthodichlorobenzène. On balaie l'autoclave avec de l'azote puis introduit 200 bars de monoxyde de carbone. Après avoir isolé le réacteur et mis en service l'agitation, on chauffe à 240 °C pendant 3 h 45 en limitant ainsi volontairement l'avancement de la réaction. Après refroidissement, l'analyse montre que le T.T.G. du nitrobenzène est de 33,1 %. On purifie le complexe en opérant de la même façon que dans l'exemple 3. On obtient ainsi la tétraphénylporphyrine de palladium avec un rendement de 78 %.

### Exemple 5

On introduit dans l'autoclave 10 g de nitrobenzène, 0,6 g de tétraphénylporphyrine et 0,26 g de $RhCl_3, 3H_2O$. On complète à 100 ml le volume total à l'aide d'orthodichlorobenzène. On balaie à l'azote et introduit 200 bars de CO. On isole l'autoclave et en maintenant une agitation, on le chauffe à 240 °C pendant 1 heure. Après refroidissement, l'analyse montre que le T.T.G. du nitrobenzène est de 100 % et la sélectivité en phénylisocyanate de 46 %. On ne détecte pas d'azobenzène.

On distille l'isocyanate et le solvant et dissout le résidu dans le volume minimum de chloroforme. On chromatographie la solution sur une colonne remplie d'alumine neutre puis évapore le solvant. On purifie le résidu par une extraction en continu à l'aide d'éther éthylique, ce qui permet d'obtenir la tétraphénylporphyrine de rhodium avec un rendement de 83 % par rapport au rhodium mis en jeu initialement.

**Revendications**

1. Procédé de synthèse des isocyanates aromatiques caractérisé en ce qu'on fait réagir un dérivé nitré aromatique avec le monoxyde de carbone en présence d'un catalyseur constitué de porphyrine métallique synthétisé *in situ* à partir d'une porphyrine et d'un métal ou d'un dérivé métallique.

2. Procédé selon la revendication 1 caractérisé en ce que le métal ou le dérivé métallique est choisi parmi les métaux ou les dérivés des métaux des groupes VIII et $I_B$ de la classification périodique des éléments.

3. Procédé selon la revendication 2 caractérisé en ce que le métal ou le dérivé métallique est le palladium, le rhodium ou un dérivé de ces métaux.

4. Procédé selon l'une des revendications 1 à 3 caractérisé en ce que le dérivé métallique se présente sous forme d'oxyde ou de sel.

5. Procédé selon la revendication 4 caractérisé en ce que le dérivé métallique est le noir de palladium ou le trichlorure de rhodium.

6. Procédé selon la revendication 1 caractérisé en ce que la porphyrine est substituée par un ou plusieurs groupements alkyles linéaires ou ramifiés comportant de un à dix atomes de carbone, ou par des groupements aromatiques comportant de six à douze atomes de carbone ou par un ou plusieurs groupes chlorure d'acide ou amides.

7. Procédé selon la revendication 6 caractérisé en ce que la porphyrine est la tétraphénylporphyrine.

8. Procédé selon l'une des revendications 1 à 7 caractérisé en ce que l'on synthétise *in situ* la tétraphénylporphyrine de palladium ou de rhodium.

9. Procédé selon l'une des revendications 1 à 8 caractérisé en ce que le dérivé nitré aromatique répond à la formule générale :

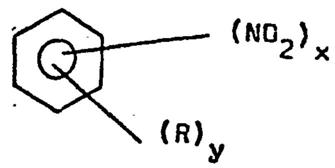

où x = 1 ou 2, y = 0, 1, 2, 3 et R représente un atome d'halogène ou un groupement alkyle ou alkoxy ayant de 1 à 10 atomes de carbone.

10. Procédé selon la revendication 9 caractérisé en ce que le dérivé nitré est le nitrobenzène ou le dinitro-2,4 toluène.

11. Procédé selon l'une des revendications 1 à 10 caractérisé en ce que le rapport du nombre d'atomes-grammes de métal sur le nombre de groupements nitro à transformer est compris entre $10^{-4}$ et 1.

12. Procédé selon l'une des revendications 1 à 11 caractérisé en ce que la réaction est menée en phase liquide en présence ou non d'un solvant.

13. Procédé selon la revendication 12 dans lequel le solvant est l'orthodichlorobenzène.

14. Procédé selon l'une des revendications 1 à 13 caractérisé en ce que la température de réaction est comprise entre 100 °C et 500 °C.

15. Procédé selon l'une des revendications 1 à 14 caractérisé en ce que la pression de réaction est comprise entre 20 et 500 bars.

**Claims**

1. Process for the synthesis of aromatic isocyanates, characterised in that an aromatic nitro derivative is reacted with carbon monoxide in the presence of a catalyst consisting of a metal porphyrin synthesised *in situ* from a porphyrin and a metal or metal derivative.

2. Process according to Claim 1, characterised in that the metal or metal derivative is chosen from amongst the metals or derivatives of the metals of groups VIII and $I_B$ of the periodic classification of the elements.

3. Process according to Claim 2, characterised in that the metal or metal derivative is palladium, rhodium or a derivative of these metals.

4. Process according to one of Claims 1 to 3, characterised in that the metal derivative is in the form of an oxide or a salt.

5. Process according to Claim 4, characterised in that the metal derivative is palladium black or rhodium trichloride.

6. Process according to Claim 1, characterised in that the porphyrin is substituted by one or more linear or branched alkyl groups containing from one to ten carbon atoms, or by aromatic groups containing from six to twelve carbon atoms, or by one or more acid chloride or amide groups.

7. Process according to Claim 6, characterised in that the porphyrin is tetraphenylporphyrin.

8. Process according to one of Claims 1 to 7, characterised in that palladium tetraphenylporphyrin or rhodium tetraphenylporphyrin is synthesised *in situ*.

9. Process according to one of Claims 1 to 8, characterised in that the aromatic nitro derivative corresponds to the general formula:

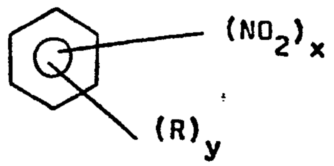

in which x = 1 or 2, y = 0, 1, 2 or 3 and R represents a halogen atom or an alkyl or alkoxy group having from 1 to 10 carbon atoms.

10. Process according to Claim 9, characterised in that the nitro derivative is nitrobenzene or 2,4-dinitrotoluene.

11. Process according to one of Claims 1 to 10, characterised in that the ratio of the number of gram atoms of metal to the number of nitro groups to be converted is between $10^{-4}$ and 1.

12. Process according to one of Claims 1 to 11, characterised in that the reaction is carried out in the liquid phase, in the presence or absence of a solvent.

13. Process according to Claim 12, in which the solvent is ortho-dichlorobenzene.

14. Process according to one of Claims 1 to 13, characterised in that the reaction temperature is between 100 °C and 500 °C.

15. Process according to one of Claims 1 to 14, characterised in that the reaction pressure is between 20 and 500 bars.

**Ansprüche**

1. Verfahren zur Synthese von aromatischen Isocyanaten, dadurch gekennzeichnet, daß man eine nitrierte, aromatische Verbindung mit Kohlenmonoxid in Gegenwart eines Katalysators aus metallischem Porphyrin zur Reaktion bringt, der *in situ* aus einem Porphyrin und einem Metall oder Metallderivat synthetisiert ist.

2. Verfahren nach Anspruch 1, dadurch gekennzeichnet, daß das Metall oder das Metallderivat aus den Metallen oder den Metallderivaten der Gruppen VIII und $I_B$ des Periodensystems der Elemente ausgewählt ist.

3. Verfahren nach Anspruch 2, dadurch gekennzeichnet, daß das Metall oder das Metallderivat Palladium, Rhodium oder ein Derivat dieser Metalle ist.

4. Verfahren nach einem der Ansprüche 1 bis 3, dadurch gekennzeichnet, daß das Metallderivat in Oxid- oder Salzform vorliegt.

5. Verfahren nach Anspruch 4, dadurch gekennzeichnet, daß das Metallderivat Palladiumschwarz oder Rhodiumtrichlorid ist.

6. Verfahren nach Anspruch 1, dadurch gekennzeichnet, daß das Porphyrin durch ein oder mehrere gerade oder verzweigte Alkylgruppen mit 1 bis 10 Kohlenstoffatomen oder durch aromatische Gruppen mit 6 bis 12 Kohlenstoffatomen oder durch eine oder mehrere Säurechlorid- oder Chloramidgruppen substituiert ist.

7. Verfahren nach Anspruch 6, dadurch gekennzeichnet, daß das Porphyrin Tetraphenylporphyrin ist.

8. Verfahren nach einem der Ansprüche 1 bis 7, dadurch gekennzeichnet, daß man das Palladium- oder Rhodiumtetraphenylporphyrin *in situ* synthetisiert.

9. Verfahren nach einem der Ansprüche 1 bis 8, dadurch gekennzeichnet, daß die nitrierte, aromatische Verbindung der allgemeinen Formel:

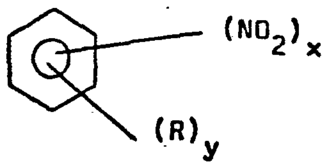

entspricht, wobei x = 1 oder 2 und y = 0, 1, 2, 3 ist und R ein Halogenatom oder eine Alkyl- oder Alkoxygruppe mit 1 bis 10 Kohlenstoffatomen darstellt.

**10.** Verfahren nach Anspruch 9, dadurch gekennzeichnet, daß die nitrierte Verbindung Nitrobenzol oder 2,4-Dinitrotoluol ist.

**11.** Verfahren nach einem der Ansprüche 1 bis 10, dadurch gekennzeichnet, daß das Verhältnis der Gramm-Atome des Metalls zur Zahl der umzuwandelnden Nitrogruppen zwischen $10^{-4}$ und 1 liegt.

**12.** Verfahren nach einem der Ansprüche 1 bis 11, dadurch gekennzeichnet, daß die Reaktion in flüssiger Phase in Gegenwart oder Abwesenheit eines Lösungsmittels durchgeführt wird.

**13.** Verfahren nach Anspruch 12, bei dem das Lösungsmittel Orthodichlorbenzol ist.

**14.** Verfahren nach einem der Ansprüche 1 bis 13, dadurch gekennzeichnet, daß die Reaktionstemperatur zwischen 100 °C und 500 °C liegt.

**15.** Verfahren nach einem der Ansprüche 1 bis 14, dadurch gekennzeichnet, daß der Reaktionsdruck zwischen 20 und 500 bar beträgt.